Europäisches Patentamt

European Patent Office

Office européen des brevets

(19)

(11) Veröffentlichungsnummer: **0 198 545**
**A2**

# (12) EUROPÄISCHE PATENTANMELDUNG

(21) Anmeldenummer: **86200604.6**

(22) Anmeldetag: **11.04.86**

(51) Int. Cl.⁴: **A 61 B 5/10**

(30) Priorität: **15.04.85 DE 3513400**

(43) Veröffentlichungstag der Anmeldung: **22.10.86**
Patentblatt 86/43

(84) Benannte Vertragsstaaten: **DE FR GB NL**

(71) Anmelder: **Philips Patentverwaltung GmbH,
Billstrasse 80, D-2000 Hamburg 28 (DE)**

(84) Benannte Vertragsstaaten: **DE**

(71) Anmelder: **N.V. Philips' Gloeilampenfabrieken,
Groenewoudseweg 1, NL-5621 BA Eindhoven (NL)**

(84) Benannte Vertragsstaaten: **FR GB NL**

(72) Erfinder: **Heizel, Thomas, Oldesloer Strasse 107,
D-2000 Hamburg 61 (DE)**
Erfinder: **Kordts, Jürgen, Pulverstrasse 11,
D-2000 Wedel (DE)**
Erfinder: **Martens, Gerhard, Dr., Hellhörn 1a,
D-2086 Ellerau (DE)**

(74) Vertreter: **Auer, Horst, Dipl.-Ing. et al, Philips
Patentverwaltung GmbH
Billstrasse 80 Postfach 10 51 49,
D-2000 Hamburg 28 (DE)**

(54) **Optischer Bewegungssensor.**

(57) Der optische Bewegungssensor dient der eindeutigen und fehlerfreien Erfassung von Formveränderungen eines menschlichen Körpers. Der Bewegungssensor besteht aus einem Gürtel mit einer aus zwei Teilen (1, 2) bestehenden Schnalle, die über einen lichtdurchlässigen Sensorkörper (4) mechanisch miteinander verbunden sind. Mit dem einen Schnallenteil (1) ist ein Sende- und ein Empfangslichtleiter (11, 12) derart verbunden, dass vom Sendelichtleiter (11) abgestrahltes Licht über einen Polarisator (13) durch den Sensorkörper (4) strahlt und über einen Analysator (14) in den Empfangslichtleiter (12) eingekoppelt wird.

EP 0 198 545 A2

ACTORUM AG

Optischer Bewegungssensor

Die Erfindung bezieht sich auf einen optischen Bewegungssensor zur Erfassung von Formveränderungen eines menschlichen Körpers.

Bei der Computertomographie, insbesondere bei der Kernspintomographie, erzeugen Darm-, Schluck- und Muskelbewegungen eines menschlichen Körpers, aber auch Bewegungen, die von dessen Atmungs- oder Herztätigkeit herrühren, sogenannte Artefakte, d.h. Schattenbilder auf den senkrecht zur Körperachse liegenden Schnittbildern, welche das Untersuchungsergebnis verfälschen können. Um fehlerfreie Schnittbilder zu erhalten, ist es somit erforderlich, Formveränderungen eines zu untersuchenden menschlichen Körpers eindeutig zu erfassen und bei der Herstellung der Schnittbilder zu berücksichtigen.

Aus der deutschen Gebrauchsmusterschrift 80 12 386 ist ein kapselförmiger Sensor zur Erfassung von Formveränderungen eines menschlichen Körpers bekannt, der als ein mit einer elastischen Membrane abgeschlossener und mit Luft gefüllter Hohlraum ausgebildet ist. Der Hohlraum weist eine Öffnung auf, die über einen Schlauch mit einem Volumenwandler verbunden ist. Der Sensor wird mittels Klebestreifen derart auf den zu untersuchenden menschlichen Körper befestigt, daß die elastische Membrane auf dem Körper aufliegt und bei Formveränderungen des Körpers auslenkt. Die dadurch verursachte Veränderung des Hohlraumvolumens wird vom Volumenwandler erfaßt und angezeigt. Da die Membrane sehr dünnwandig ist, wird die im Sensor befindliche Luft erwärmt. Diese dehnt sich aus, so daß die vom Volumenwandler gelieferten Meßergebnisse einen von der Temperatur des kapselförmigen Sensors abhängigen Meßfehler aufweisen. Die Meßergebnisse sind somit sowohl von der Form des zu untersuchenden Körpers als auch von der Temperatur des Sensors abhängig und können deshalb keine eindeutige Auskunft über die Verformungszustände des Körpers geben.

Aufgabe der Erfindung ist es, einen optischen Bewegungssensor zu schaffen, der von der Körpertemperatur unabhängige, eindeutige Aussagen über die Verformungszustände des zu untersuchenden menschlichen Körpers zu bestimmten Zeitpunkten liefert.

Diese Aufgabe wird bei einem optischen Bewegungssensor eingangs genannter Art durch einen um den Körper eines Patienten herumzulegenden Gürtel gelöst, der eine aus zwei Teilen bestehende Schnalle aufweist, die über einen lichtdurchlässigen Sensorkörper mechanisch miteinander verbunden sind, wobei im Bereich eines Schnallenteils ein Sende- und ein Empfangslichtleiter über einen Polarisator bzw. einen Analysator mit dem Sensorkörper derart gekoppelt sind, daß vom Sendelichtleiter abgestrahltes Licht über den Polarisator durch den Sensorkörper hindurchgeht und dann über den Analysator in den Empfangslichtleiter gelangt.

Verformt sich der menschliche Körper, verändert sich der Spannungszustand des Gürtels und damit auch des lichtdurchlässigen Sensorkörpers, wodurch der Polarisationszustand von polarisiertem Licht beim Durchgang durch den Sensorkörper verändert wird. Ein Analysator filtert aus dem Licht mit spannungsabhängigem Polarisationszustand linear polarisiertes Licht aus, dessen Intensität sich mit dem Polarisationszustand des Lichtes verändert und somit

0198545

weitgehend temperaturunabhängige und eindeutige Aussagen
über den augenblicklichen Spannungszustand des Gürtels und
damit über den Verformungszustand des Körpers liefert.

Ein einfach und preisgünstig herstellbarer Sensor ergibt
sich, wenn der Empfangslichtleiter auf der dem Sendelichtleiter abgewandten Seite des Sensorkörpers angeordnet ist.

Um die Empfindlichkeit des Bewegungssensors zu vergrößern,
ist es vorteilhaft, wenn der Sende- und der Empfangslichtleiter auf derselben Seite des Sensorkörpers angeordnet
sind und wenn auf der den Lichtleitern abgewandten Seite
des Sensorkörpers ein Spiegel angeordnet ist, der das vom
Sendelichtleiter abgestrahlte und durch den Sensorkörper
hindurchgehende Licht durch den Sensorkörper wieder
zurückreflektiert und in den Empfangslichtleiter einkoppelt. Hierbei durchstrahlt das Licht den Sensorkörper
zweimal, so daß auch der Polarisationszustand des Lichtes
in Abhängigkeit des Spannungszustandes des Bewegungssensors zweimal gleichsinnig verändert wird.

In einer vorteilhaften Ausgestaltung des Bewegungssensors
weist das eine Schnallenteil eine Steckerbuchse für einen
mit dem Sende- und dem Empfangslichtleiter verbundenen
Lichtleitfaserstecker auf, der eine zwischen den Stirnflächen der Lichtleiter und dem Sensorkörper angeordnete
Polarisationsfolie enthält, die für das vom Sendelichtleiter abgestrahlte Licht als Polarisator und für das vom
Spiegel in den Empfangslichtleiter einzukoppelnde Licht
als Analysator wirkt. Hierbei ist lediglich ein einziger
Lichtleitfaserstecker erforderlich, um den Sende- und den
Empfangslichtleiter mit dem Bewegungssensor zu verbinden.
Außerdem können beide Lichtleiter in einem Lichtleiterkabel untergebracht werden, so daß Patienten durch einzelne
Lichtleiter nicht so sehr belästigt werden.

Um bei der Kernspintomographie Meßfehler aufgrund von magnetischen Feldern zu vermeiden, die von Wirbelströmen in elektrisch leitfähigen Teilen des Bewegungssensors verursacht werden, ist es notwendig, daß der optische Bewegungssensor aus nicht-metallischen Werkstoffen hergestellt ist.

Anhand der Zeichnungen werden einige Ausführungsbeispiele der Erfindung beschrieben und deren Wirkungsweise erläutert. Es zeigen

Fig. 1 eine Seitenansicht der zweiteiligen Schnalle im Schnitt entlang der Linie I-I in Fig. 2 und 3,

Fig. 2 ein Schnittbild der Schnalle entlang der Linie II-II in Fig. 1,

Fig. 3 ein Schnittbild der Schnalle entlang der Linie III-III in Fig. 1,

Fig. 4 eine Seitenansicht eines Teiles der Schnalle, bei welcher der Sende- und der Empfangslichtleiter in einem Lichtleitfaserstecker untergebracht sind.

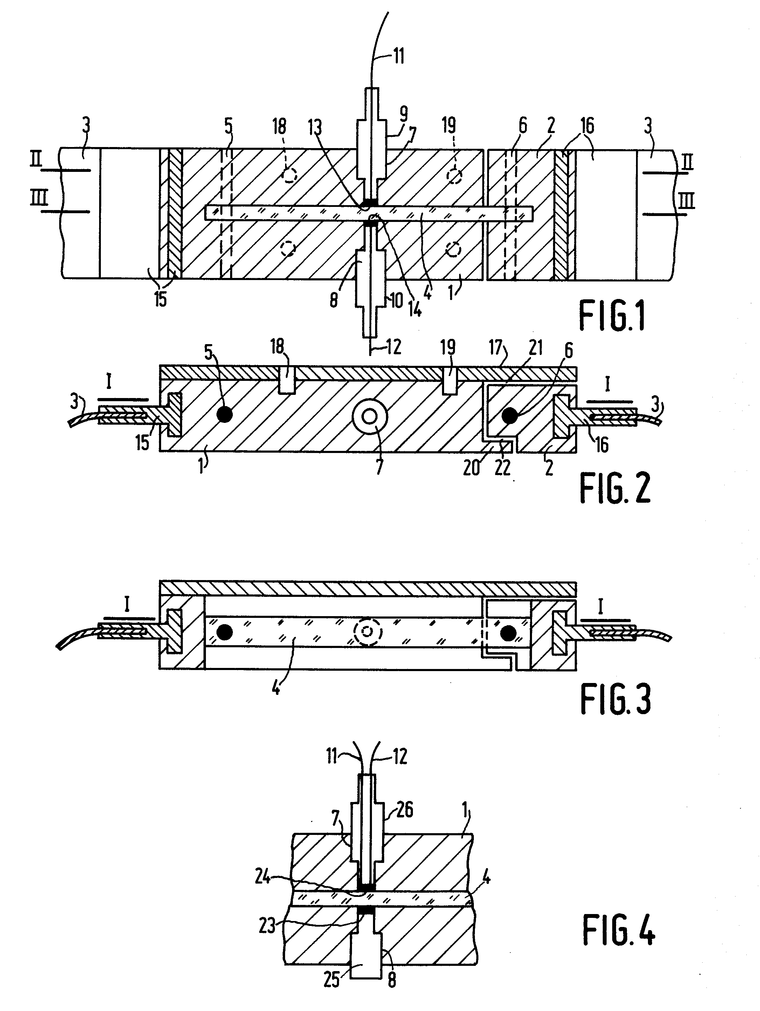

In Fig. 1 ist die aus zwei Teilen 1 und 2 bestehende Schnalle im Schnitt dargestellt. Die beiden Schnallenteile 1 und 2 sind mit je einem Ende eines Gurtriemens 3 mechanisch verbunden, der um den Oberkörper oder Bauch eines zu untersuchenden Patienten gelegt wird. Außerdem sind die beiden Schnallenteilen 1 und 2 über einen als längliche Platte ausgebildeten, lichtdurchlässigen Sensorkörper 4 mechanisch miteinander verbunden. Hierbei ist der Sensorkörper 4 mittels Splinte 5 und 6 an den Schnallenteilen 1 und 2 befestigt.

0198545
PHD 85-056 EP

Das eine Schnallenteil 1 weist beidseitig des Sensorkörpers 4 je eine Steckerbuchse 7 und 8 auf, in die je ein Lichtleitfaserstecker 9 und 10 für einen Sende- und einen Empfangslichtleiter 11 und 12 einsteckbar sind. Die Lichtleitfaserstecker 9 und 10 enthalten zwischen den Stirnflächen des Sende- und des Empfangslichtleiters 11 und 12 und dem Sensorkörper 4 je eine Polarisationsfolie 13 und 14, die im Lichtleitfaserstecker 9 als Polarisator und im Lichtleitfaserstecker 10 als Analysator wirken. Die Durchlaßrichtungen des Polarisators und des Analysators liegen parallel zueinander und schließen mit der Längsachse des Sensorkörpers 4 einen Winkel von 45° ein.

Der gesamte optische Bewegungssensor ist aus nicht-metallischen Werkstoffen hergestellt. So können der Gurtriemen 3 aus Leder, die beiden Schnallenteile 1 und 2, die Splinte 5 und 6 und die Lichtleitfaserstecker 9 und 10 aus Kunststoff und der Sensorkörper 4 aus Glas oder aus durchsichtigem Kunststoff gefertigt sein.

In Fig. 2 ist ein Schnittbild entlang der Linie II-II in Fig. 1 dargestellt. Die beiden Enden des Gurtriemens 3 sind mit je einer T-förmigen Halterung 15 und 16 verbunden, die, wie auch Fig. 1 zeigt, in die beiden Schnallenteile 1 und 2 einschiebbar sind. In Fig. 2 sind die beiden Splinte 5 und 6 und die Steckerbuchse 7 im Schnitt dargestellt. Weiterhin zeigt Fig. 2, daß die Vorderseite der Schnalle von einer Platte 17 abgedeckt ist, die mittels Kunststoffschrauben 18 und 19 an dem Schnallenteil 1 befestigt ist. Die Platte 17 und ein Vorsprung 20 des Schnallenteils 1 bilden für das Schnallenteil 2 eine Führung, das hierdurch über Silikonscheiben 21 und 22 gegen seitliches Auslenken gesichert ist. Außerdem werden durch die elastischen Silikonscheiben 21 und 22 Haft- und Gleitreibungsverluste aufgrund der Relativbewegungen der beiden Schnallenteile 1 und 2 vermieden.

Fig. 3 zeigt ein Schnittbild der Schnalle entlang der Linie III-III in Fig. 1, in dem eine mögliche Ausgestaltung des Sensorkörpers 4 dargestellt ist. Sowohl die Breite als auch die Dicke des plattenförmigen Sensorkörpers 4 sind hierbei von der geforderten Empfindlichkeit des optischen Bewegungssensors abhängig. Auch die Wahl des Werkstoffes hat Auswirkungen auf die Verformbarkeit und damit auf die Empfindlichkeit des Sensorkörpers 4. Ein Bewegungssensor mit einem Sensorkörper 4 aus lichtdurchlässigem Kunststoff weist eine größere Empfindlichkeit auf als ein Bewegungssensor mit einem Sensorkörper 4 aus Glas, da Kunststoff leichter verformbar als Glas ist.

In Fig. 4 ist ein Teil der Schnalle im Schnitt dargestellt, bei welcher der Sende- und der Empfangslichtleiter 11 und 12 auf einer Seite des Sensorkörpers 4 angeordnet sind. Der Sende- und der Empfangslichtleiter 11 und 12 sind hierbei über einen einzigen Lichtleitfaserstecker 26 mit dem Schnallenteil 1 verbunden. Auf der den Stirnflächen von Sende- und Empfangslichtleiter 11 und 12 gegenüberliegenden Seite des Sensorkörpers 4 ist ein Spiegel 23 angeordnet, der beispielsweise als dielektrischer Vielschichtspiegel ausgebildet sein kann, der aus mehreren dünnen, durchsichtigen, abwechselnd hoch- und niedrigbrechenden $\lambda/4$-Schichten besteht. Derartige Spiegel können ein Reflexionsvermögen von ca. 99,9 % haben. Der Lichtleitfaserstecker 26 weist zwischen den Stirnflächen von Sende- und Empfangslichtleiter 11 und 12 und dem Sensorkörper 4 eine Polarisationsfolie 24 auf, die für das vom Sendelichtleiter 11 abgestrahlte Licht als Polarisator und für das vom Spiegel 23 in den Empfangslichtleiter 12 eingekoppelte Licht als Analysator wirkt. Die Durchlaßrichtung der Polarisationsfolie 24 schließt mit der Längsachse des Sensorkörpers 4 einen Winkel von 45° ein. Der Spiegel 23 kann hierbei am Boden eines Blindsteckers 25 angeordnet sein, der denselben Querschnitt wie ein Lichtleitfaserstecker aufweist und der somit in die Steckerbuchse 8 einsteckbar ist.

Der Spannungszustand des um den Oberkörper oder Bauch eines Patienten gelegten Gürtels wird von jeder Formveränderung aufgrund der Herz- und Atmungstätigkeit, aber auch aufgrund von Darm-, Schluck- und Muskelbewegungen verändert, so daß sich dadurch auch die auf den Sensorkörper 4 einwirkende Zugspannung verändert. Das vom Sendelichtleiter 11 abgestrahlte und vom Polarisator 13 oder 24 linear polarisierte Licht wird umso mehr elliptisch polarisiert, je größer die auf den Sensorkörper 4 einwirkende Zugspannung ist. Nach Durchstrahlen des Sensorkörpers 4 wird vom Analysator 14 aus dem elliptisch polarisierten Licht linear polarisiertes Licht ausgefiltert, dessen Intensität bei parallel liegenden Durchlaßrichtungen von Polarisator 13 und Analysator 14 umso geringer ist, je mehr das Licht im Sensorkörper 4 elliptisch polarisiert wurde, d.h. je mehr Zugspannung vom Gürtel auf den Sensorkörper 4 übertragen wird. Die Intensität des in den Empfangslichtleiter 12 eingekoppelten Lichtes gibt somit Auskunft über die Zugspannung des gürtelförmigen Bewegungssensors und damit über Veränderungen des Umfanges des zu untersuchenden menschlichen Körpers.

Bei dem in Fig. 4 dargstellten Ausführungsbeispiel wird das vom Sendelichtleiter 11 abgestrahlte Licht durch die zunächst als Polarisator wirkende Polarisationsfolie 24 linear polarisiert. Das linear polarisierte Licht strahlt durch den Sensorkörper 4 und wird dabei in Abhängigkeit der auf den Sensorkörper 4 einwirkenden Zugspannung elliptisch polarisiert. Dieses elliptisch polarisierte Licht wird vom Spiegel 23 durch den Sensorkörper 4 zurückgestrahlt, der das Licht zusätzlicht derart elliptisch polarisiert, daß die große Achse der Polarisationsellipse verkleinert und die kleine Achse vergrößert wird. Danach passiert das Licht dieselbe nun als Analysator wirkende Polarisationsfolie 24 und wird in den Empfangslichtleiter 12 eingekoppelt, wobei der Analysator aus dem elliptisch polarisierten Licht linear polarisiertes Licht ausfiltert, dessen Intensität mit einer Zunahme der auf den Sensorkörper 4 einwirkenden Zugspannung sehr stark abnimmt, so daß über die Messung dieser Intensität bereits kleine Formveränderungen des zu untersuchenden menschlichen Körpers erfaßbar sind.

## PATENTANSPRÜCHE

1.    Optischer Bewegungssensor zur Erfassung von Formveränderungen eines menschlichen Körpers, gekennzeichnet durch einen um den Körper eines Patienten herumzulegenden Gürtel (3), der eine aus zwei Teilen (1,2) bestehende Schnalle aufweist, die über einen lichtdurchlässigen Sensorkörper (4) mechanisch miteinander verbunden sind, wobei im Bereich eines Schnallenteils (1) ein Sende- und ein Empfangslichtleiter (11,12) über einen Polarisator (13) bzw. einen Analysator (14) mit dem Sensorkörper (4) derart gekoppelt sind, daß vom Sendelichtleiter (11) abgestrahltes Licht über den  Polarisator (13) durch den Sensorkörper (4) hindurchgeht   und dann über den Analysator (14) in den Empfangslichtleiter (12) gelangt.

2.    Optischer Bewegungssensor nach Anspruch 1, dadurch gekennzeichnet, daß der Empfangslichtleiter (12) auf der dem Sendelichtleiter (11) abgewandten Seite des Sensorkörpers (4) angeordnet ist.

3.    Optischer Bewegungssensor nach Anspruch 1, dadurch gekennzeichnet, daß der Sende- und der Empfangslichtleiter (11,12) auf derselben Seite des Sensorkörpers (4) angeordnet sind und daß auf der den Lichtleitern (11,12)abgewandten Seite des Sensorkörpers (4) ein Spiegel (23) angeordnet ist, der das vom Sendelichtleiter (11) abgestrahlte und durch den Sensorkörper (4) hindurchgehende Licht durch den Sensorkörper (4) zurückreflektiert und in den Empfangslichtleiter (12) einkoppelt.

0198545

4.     Optischer Bewegungssensor nach Anspruch 3, dadurch gekennzeichnet, daß das eine Schnallenteil (1) eine Steckerbuchse (7) für einen mit dem Sende- und dem Empfangslichtleiter (11,12) verbundenen Lichtleitfaserstecker (26) aufweist, der eine zwischen den Stirnflächen der Lichtleiter (11,12) und dem Sensorkörper (4) angeordnete Polarisationsfolie (24) enthält, die für das vom Sendelichtleiter (11) abgestrahlte Licht als Polarisator und für das vom Spiegel (23) in den Empfangslichtleiter (12) einzukoppelnde Licht als Analysator wirkt.

5.     Optischer Bewegungssensor nach Anspruch 1 bis 4, dadurch gekennzeichent, daß die Einzelteile des optischen Bewegungssensors aus nicht-metallischen Werkstoffen hergestellt sind.

1/1                 0198545

**FIG.1**

**FIG.2**

**FIG.3**

**FIG.4**